(19) Europäisches Patentamt
European Patent Office
Office européen des brevets

(11) Veröffentlichungsnummer: **0 015 541**
**B1**

(12) **EUROPÄISCHE PATENTSCHRIFT**

(45) Veröffentlichungstag der Patentschrift: **07.03.84**

(51) Int. Cl.³: **C 07 F 1/02,** C 01 B 6/04, B 01 J 31/16

(21) Anmeldenummer: **80101095.0**

(22) Anmeldetag: **05.03.80**

(54) **Verfahren zur Herstellung von Organolithiumverbindungen neben Lithiumhydrid; Katalysatoren.**

(30) Priorität: **07.03.79 DE 2908928**

(43) Veröffentlichungstag der Anmeldung: **17.09.80 Patentblatt 80/19**

(45) Bekanntmachung des Hinweises auf die Patenterteilung: **07.03.84 Patentblatt 84/10**

(84) Benannte Vertragsstaaten: **AT BE CH DE FR GB IT LU NL SE**

(56) Entgegenhaltungen:
**CH - A - 585 760**
**DE - A - 2 722 221**
**DE - B - 1 150 679**
**DE - B - 1 177 157**

(73) Patentinhaber: **Studiengesellschaft Kohle mbH, Kaiser-Wilhelm-Platz 1, D-4330 Mülheim/Ruhr (DE)**

(72) Erfinder: **Bogdanovic, Borislav, Dr., Kaiser-Wilhelm-Platz 1, D-4330 Mülheim/Ruhr (DE)**

(74) Vertreter: **von Kreisler, Alek, Dipl.-Chem. et al, Deichmannhaus am Hauptbahnhof, D-5000 Köln 1 (DE)**

EP 0 015 541 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Verfahren zur Herstellung von Organolithiumverbindungen neben Lithiumhydrid; Katalysatoren

Die Erfindung betrifft ein katalytisches Verfahren zur Herstellung von Organolithiumverbindungen aus Lithium und Olefinen, wobei als weiteres Reaktionsprodukt eine äquimolare Menge Lithiumhydrid anfällt.

Die herkömmliche technische Methode zur Herstellung von Organolithiumverbindungen (Kirk-Othmer, «Enc. Chem. Tech.», Vol. 12, S. 547, 1967) beruht auf der Reaktion von Lithiummetall mit organischen Halogenverbindungen bei der Organolithiumverbindungen neben Lithiumhalogeniden entstehen:

$$RX + 2\,Li \rightarrow RLi + LiX \qquad (1)$$

$X = Cl, Br, J$

Allyl- bzw. Benzyllithiumverbindungen lassen sich u.a. durch die Spaltung der entsprechenden Äther- bzw. Acyloxyderivate mit Lithiummetall darstellen (J.A. Katzenellenbogen, R.S. Lenox, J. Org. Chem., 38, 326 [1973]; U. Schöllkopf in «Methoden der Organischen Chemie», Houben-Weyl, XIII/1, S. 161; J.J. Eisch, A.M. Jacobs, J. Org. Chem., 28, 2145 [1973]):

$$ROR' + 2\,Li \rightarrow R\text{–}Li + R'OLi \qquad (2)$$

$R = $ Allyl, Benzyl
$R' = $ Phenyl, Mesitoyl

Aus den so hergestellten Organolithiumverbindungen lassen sich zahlreiche weitere Organolithiumverbindungen durch Metall-H-Austausch:

$$R\text{–}Li + R'\text{–}H \rightarrow R\text{–}H + R'\text{–}Li \qquad (3)$$

bzw. Metall-Halogen-Austausch (D. Seebach. K.-H. Geiss in «New Applications of Organometallic Reagents in Organic Synthesis», S. 1, Elsevier, 1976):

$$R\text{–}Li + R'\text{–}X \rightarrow R'\text{–}Li + R\text{–}X \qquad (4)$$

$X = Cl, Br, J$

darstellen.

Nur in Ausnahmefällen war es bisher möglich, Organolithiumverbindungen unmittelbar aus Lithiummetall und Kohlenwasserstoffen zu synthetisieren. So lassen sich z.B. 1-Alkine (H. Ogura, H. Takahashi, Synth. Commun., 3, 135 [1973]), Triphenylmethan oder Acenaphtylen (B.J. Wakefield, «The Chemistry of Organolithium Compounds», S. 70, Pergamon Press, 1974), mit Lithiummetall lithiieren. Nach D.L. Skinner et al. (J. Org. Chem., 32, 105 [1967]) reagiert Lithium mit 1-Alkinen in Abwesenheit eines Lösungsmittels zu 1-Alkinyllithiumverbindungen und Lithiumhydrid:

$$RCH{\equiv}CH_2 + 4\,Li \rightarrow RC{\equiv}C\text{–}Li + 3\,LiH \qquad (5)$$

wobei als Nebenprodukte der Reaktion in sehr kleinen Ausbeuten 1-Lithio-1-alkene auftreten. In Gegenwart von Tetrahydrofuran –THF– wurde aus Lithium und 1-Hexen bei Siedetemperatur 1-Lithio-1-hexen in 9% Ausbeute erhalten.

Ein Verfahren zur Herstellung von Organolithiumverbindungen aus Lithium und Äthylen in Dimethoxymethan oder THF in Gegenwart von Biphenyl und gegebenenfalls Naphthalin wurde kürzlich bekannt (V. Rautenstrauch [Firmenich S.A., Genf], Schweiz. Pat. 585 760, 20.05.1974; V. Rautenstrauch, Angew. Chem., 87, 254 [1975]).

Die Ausbeuten an Organolithiumverbindungen nach diesem Verfahren liegen äusserst niedrig. Da die Reaktionsprodukte ausserdem in Form eines Gemisches von Vinyllithium und 1,4-Dilithiobutan anfallen, erscheint dieses Verfahren für technische Zwecke kaum geeignet.

Es wurde nun überraschenderweise gefunden, dass man α-Olefine und α,ω-Diolefine mit metallischem Lithium in Gegenwart geeigneter Katalysatoren zur Reaktion bringen kann, wobei als Reaktionsprodukte weitgehend einheitliche Organolithiumverbindungen neben Lithiumhydrid entstehen. Die Reaktion zwischen Lithium und Olefinen wird zweckmässigerweise in einem cyclischen oder offenkettigen Mono- oder Polyäther – bevorzugt in Tetrahydrofuran (THF) als Lösungsmittel – bei Temperaturen zwischen −100 und +100 °C – vorzugsweise zwischen −20 und +50 °C und bei Partialdrucken unter 1 sowie bei 1 bis 100 bar Druck durchgeführt.

Die Erfindung betrifft dementsprechend ein Verfahren zur Herstellung von Organolithiumverbindungen neben Lithiumhydrid, das dadurch gekennzeichnet ist, dass man

a) Lithium in Gegenwart eines Katalysators, hergestellt aus polycyclischen Aromaten wie Anthracen, Naphthalin und Biphenyl und einem Alkalimetall in Gegenwart einer Übergangs-Metallverbindung von Metallen der I., II., IV., V., VI., VII. und VIII. Nebengruppe des Periodensystems, oder
b) Lithium in Gegenwart eines Katalysators aus einer Mischung von Hetero-Verbindungen der allgemeinen Formeln I bis V

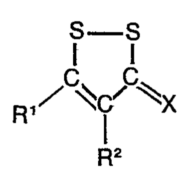
I

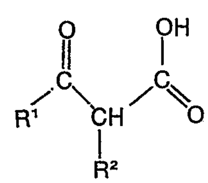
II

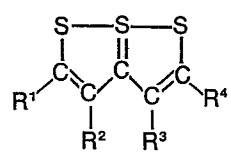
III

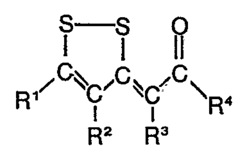
IV

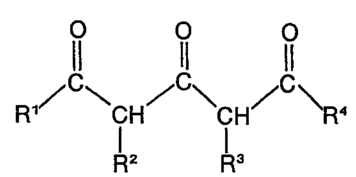
V

in denen X = Schwefel oder Sauerstoff, $R^1$, $R^2$, $R^3$ und $R^4$ Wasserstoff, Alkyl-, Cycloalkyl, Aralkyl- oder Arylreste bedeuten und/oder zwei oder mehrere solcher Reste zu einem aliphatischen oder aromatischen Ringsystem geschlossen sind, mit einer Übergangsmetallverbindung von Metallen der I., II., IV., V., VI., VI. und VIII. Nebengruppe des Periodensystems in einem Lösungsmittel mit einem α-Olefin oder α,ω-Diolefin umsetzt.

Die Erfindung betrifft ferner Katalysatoren aus

a) Komplexen von polycyclischen Aromaten wie Anthracen, Naphthalin und Biphenyl und einem Alkalimetall mit
b) einer Übergangs-Metallverbindung von Metallen der I., II., IV., V., VI., VII. und VIII. Nebengruppe des Periodensystems.

Als Arylreste gelten dabei aromatische Reste wie Phenyl-, Naphthyl-, Anthryl-, Phenanthryl-, Tolyl- oder ähnliche, als Aralkylreste durch Aryl-Gruppen substituierte Alkylreste, wie Benzyl-, Phenylethyl- oder ähnliche Reste.

Als Metalle der I., II., IV., V., VI., VII. und VIII. Nebengruppe des Periodensystems können u.a. beispielsweise Kupfer, Gold, Zink, Cadmium, Titan, Zirkon, Vanadin, Niob, Tantal, Chrom, Molybdän, Wolfram, Mangan, Eisen, Kobalt, Nickel, Ruthenium, Rhodium, Palladium, Osmium, Iridium und Platin genannt werden. Von diesen werden z.Zt. Kupfer, Eisen, Zink, Palladium, Platin oder Rhodium bevorzugt.

Die Katalysatorbildung kann auch in der Weise erfolgen, dass man die Verbindungen der allgemeinen Formeln III, IV, V, VI und VII – die auch in der DE-OS 2 722 221.5 beschrieben sind – mit Alkalimetallen, insbesondere Lithium und gegebenenfalls mit einer Metallverbindung von Metallen der I., II., IV., V., VI., VII. und VIII. Nebengruppe des Periodensystems in einem geeigneten Lösungsmittel, ggf. in Gegenwart des α-Olefins oder α,ω-Diolefins mischt. Ein besonders aktives und selektiv wirkendes Katalysatorsystem im Sinne des vorliegenden Verfahrens entsteht, wenn 2,5-Diphenyl-1,6,6a-trithiapentalen (V, $R^1 = R^4 = C_6H_5$, $R^2 = R^3 = H$) in Kombination mit Zinkchlorid in Gegenwart von α-Olefinen bzw. α,ω-Diolefinen in THF mit Lithium umgesetzt wird (vergleiche Beispiele 40–42).

Schliesslich kann man auch isolierbare Addukte zwischen Verbindungen der oben unter b) angegebenen allg. Formeln III–VII und Übergangsmetallverbindungen der I., II., IV., V., VI., VII. und VIII. Nebengruppe des Periodensystems als Katalysatoren auf das Lithium und Olefin bzw. Diolefin einwirken lassen. So bilden z.B. Eisen(III)-, Kupfer(I)- und -(II) sowie Molybdän (V)-chlorid mit 1,2-Dithiol-3-thionen bzw. 1,6,6a-Trithiapentalenen 2:1 Addukte, die anstelle eines Gemisches beider Komponenten zur Herstellung der Katalysatoren eingesetzt werden können. Auch der aus 2,5-Diphenyl-1,6,6a-Trithiapentalen und $PdCl_2$ herstellbare Komplex

ortho-Chlorpalladio-2,5-diphenyl-1,6,6a-trithiapentalen

(6) ergibt mit Lithium in THF einen aktiven Katalysator für die Lithiierung von Olefinen:

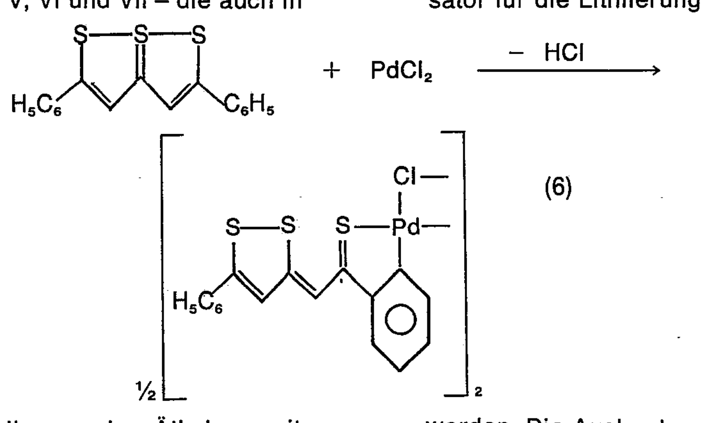


(6)

Die katalytische Lithiierung des Äthylens mit Hilfe der erfindungsgemässen Katalysatoren, in z.B. THF, führt zu Vinyllithium und Lithiumhydrid;

$$CH_2 = CH_2 + 2\,Li \xrightarrow{Kat./THF} CH_2 = CHLi + LiH \quad (7)$$

Das in THF lösliche Vinyllithium kann von dem unlöslichen Lithiumhydrid getrennt und in Lösung weiterverwendet oder in kristalliner Form isoliert werden. Die Ausbeuten an Vinyllithium liegen, je nach Katalysator, bei 60 bis über 70% der nach (7) berechneten Menge.

Bei der katalytischen Lithiierung des Propens nach dem erfindungsgemässen Verfahren entstehen im allgemeinen vier isomere Organolithiumverbindungen: trans-1-propenyllithium (9), cis-1-Propenyllithium (10), Isopropenyllithium (11) und Allyllithium (12) neben Lithiumhydrid:

$$CH_2 = CHCH_3 + 2\,Li \xrightarrow{Kat./THF}$$

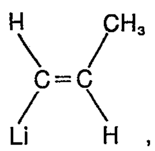
,
9

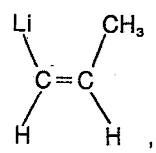
,
10

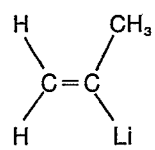
,
11

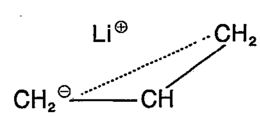
12

+ LiH (8)

Die Selektivität dieser Reaktion in bezug auf die Bildung einzelner Isomerer lässt sich durch die Wahl des Katalysators steuern. So entsteht in Gegenwart des Katalysatoren hergestellt unter Verwendung von Eisen-, Kupfer-, Kobalt- bzw. Zinkverbindungen mit einer hohen Selektivität das trans-1-Propenyllithium 9. Dahingegen lässt sich mit Hilfe der Katalysatoren hergestellt unter Verwendung von Palladium-, Platin- bzw. Rhodiumverbindungen die katalytische Lithiierung des Propens so steuern, dass überwiegend das Allyllithium 12 entsteht. Als ein besonderer in diesem Sinne selektiv wirkender Katalysator erweist sich der Palladium-Komplex (6), mit dessen Hilfe Allyllithium mit einer Selektivität von 85–90% erhalten werden kann. Am Beispiel der Lithiierung von 1-Buten mit diesem Palladium-Komplex als Katalysator wird gezeigt, dass auch höhere α-Olefine selektiv in der Allyl-Position lithiiert werden können. Anderseits lassen sich mit Hilfe der Katalysatoren, hergestellt unter Verwendung von Zink-, Eisen- bzw. Kupferverbindungen, auch höhere 1-Alkene wie 1-Buten, 1-Penten, 1-Okten und 1,7-Oktadien selektiv in der trans-1-Stellung lithiieren. So z.B. lässt sich 1-Okten mit Hilfe des oben erwähnten Katalysators aus 2,5-Diphenyl-1,6,6a-trithiapentalen und $ZnCl_2$ mit einer Selektivität von > 96% in der 1-trans-Stellung lithiieren.

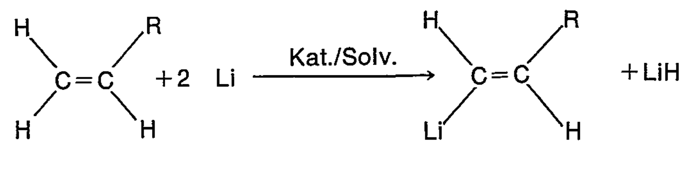


(9)

$R = CH_3, C_2H_5, n\text{–}C_3H_7, n\text{–}C_6H_{11}, -(CH_2)_n-$ usw.

Die trans-1-Lithio-1-alkene lassen sich ggf. in analysenreiner, kristalliner Form isolieren. Durch Kristallisation wird im allgemeinen der Anteil an trans-1-Lithio-1-alken erhöht. Das vorliegende Verfahren ermöglicht somit eine selektive Herstellung von trans-1-Alkenyl- bzw. Allyllithiumverbindungen aus α-Olefinen bzw. Diolefinen und Lithium.

Bei der katalytischen Lithiierung von 1,4-Pentadien in Gegenwart des 4,5-Benzo-1,2-dithiol-3-thion·$2CuCl_2$-Komplexes entsteht eine bisher unbekannte Organolithiumverbindung mit der Struktur:

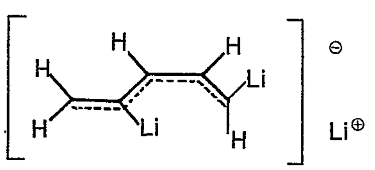

Ausgangsprodukte für die Herstellung von Organolithiumverbindungen gemäss vorliegender Erfindung sind α-Olefine der allgemeinen Formel $CH_2{=}CHR$, in der R = H, Alkyl, Aryl, Cycloalkyl oder Aralkyl sein kann, oder Diolefine der allgemeinen Formel $CH_2{=}CH\text{–}(CHR)_n\text{–}CH{=}CH_2$, in der R die gleiche Bedeutung wie oben hat und n = 1–6 ist.

Die katalytische Lithiierung von α-Olefinen bzw. α,ω-Diolefinen gemäss der Erfindung stellt eine neue Herstellungsmethode dar zur Herstellung lithiumorganischer Verbindungen, die auf anderem Wege nicht oder schwierig zugänglich sind. Anstelle der teuereren und teils giftigen sowie meist schwierig zugänglichen Organohalogenverbindungen benutzt das vorliegende Verfahren handelsübliche Olefine. Darüber hinaus fällt bei Anwendung der herkömmlichen Methode die Hälfte des eingesetzten Lithiums in Form von Lithiumhalogenid an und geht damit für weitere Umsetzungen verloren. Das erfindungsgemässe Verfahren liefert neben der Organolithiumverbindung hoch reaktives, technisch wertvolles Lithiumhydrid. Das gesamte eingesetzte Lithium wird zu hochwertigen Lithiumverbindungen umgesetzt.

Das vorliegende Verfahren gestattet eine regio- bzw. stereoselektive Synthese von Organolithiumverbindungen, wobei die Möglichkeit gegeben ist, durch geeignete Wahl des Katalysators bzw. der Reaktionsbedingungen die Reaktion so zu steuern, dass je nach Bedarf aus einem Ausgangsolefin verschiedene Organolithiumverbindungen erhalten werden können.

Die nach dem vorliegenden Verfahren herstellbaren Organolithiumverbindungen können als Initiatoren für anionische Polymerisationen von Mono- bzw. Diolefinen, bzw. als Reagenzien zur Einführung von organischen, ungesättigten Resten sowie zur Reduktion in der organischen Synthese angewendet werden.

Die Erfindung wird in den Beispielen erläutert.

## Beispiele

Sämtliche Versuche zur Darstellung lithiumorganischer Verbindungen werden in einer Schutzgasatmosphäre (z.B. Argon) durchgeführt.

### Beispiel 1

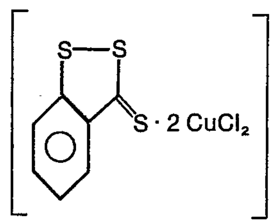

13

Zur Darstellung des 4,5-Benzo-1,2-dithiol-3-thion·$2CuCl_2$-Komplexes (13) werden 2,83 g (21,05 mMol) wasserfreies Kupfer(II)-chlorid in 100 ml Benzol suspendiert, mit 2,00 g (10,85 mMol) 4,5-Benzo-1,2-dithiol-3-thion versetzt und die Mischung 18 Std. bei Raumtemperatur gerührt. Die Suspension wird filtriert, der Niederschlag mit Benzol gewaschen und bei $1,33 \times 10^{-3}$ mbar getrocknet. Man erhält 3,58 g (75% d. Th.) des Komplexes 13. $C_7H_4S_3Cu_2Cl_4$ (453,16);

ber. C 18,55 H 0,89 S 21,22 Cu 28,04 Cl 31,29
gef. C 17,50 H 1,00 S 20,90 Cu 27,60 Cl 32,70.

Eine Lösung von 1,40 g (3,09 mMol) des Komplexes 13 in 100 ml abs. THF wird bei 0 °C mit Propen (1 bar) gesättigt und anschliessend wird der Lösung in Propenatmosphäre bei 0 °C und unter Rühren 5,07 g (0,73 Mol) Lithiumsand zugegeben (Molverh. 13:Li = 1:236). Nach einem vorübergehenden Temperaturanstieg beginnt nach 10–15 Min. die Propenaufnahme, deren Geschwindigkeit mit Hilfe einer an das Reaktionsgefäss angeschlossenen Gasbürette gemessen wird. Während der Propenaufnahme wird die Suspension gerührt, der Propendruck bei 1,1–1,2 bar und die Temperatur bei 0 –+2 °C gehalten. Das dunkelbraune Reaktionsgemisch nimmt bis zur Sättigung innerhalb von 49 Std. 6,0 l Propen (1 bar, 20 °C) auf (68,5% d. Th.). Die Suspension wird bei 0 °C filtriert, der Niederschlag mit THF gewaschen und bei 0,27 mbar getrocknet. Man erhält 4,41 Lithiumhydrid im Gemisch mit wenig Lithium (0,135 g des Gemisches liefern mit $D_2O$ 257 ml Gas (1 bar, 20 °C), bestehend aus HD (70%), $D_2$ (19%) und $H_2$ (11%). Zur Analyse der in der Lösung befindlichen Organolithiumverbindungen wird ein aliquoter Teil der Lösung (8,0 ml von insgesamt 142,0 ml) im Vakuum (0,2 Torr) eingedampft und der feste Rückstand hydrolysiert; die dabei entwickelte Gasmenge beträgt 335,5 ml (1 bar, 20 °C) und besteht aus Propen (84,9%), THF (4,6%), $H_2$ (3,5%) und Acetylen (1,4%). Aus der Propenmenge errechnet sich nach Gl. 8 eine Ausbeute an Organolithiumverbindungen $LiC_3H_5$ von 57,7%. Zur Bestimmung der Isomerenverteilung werden 58,0 ml der Lösung im Vakuum (0,2 Torr) eingedampft, der Rückstand in 60 ml Äther gelöst, bei 0 °C mit 18,9 g (174 mMol) Trimethylchlorsilan versetzt und das Gemisch 12 Std. bei 20 °C gerührt. Die Hydrolyse bzw. Aufarbeitung und Destillation liefert neben Hexamethyldisiloxan 7,3 g eines Gemisches der isomeren Silane $(CH_3)_3\ SiC_3H_5$ (Sdp. 87–89 °C/1,01 bar), bestehend aus trans-1-Propenyltrimethylsilan 74,5%, cis-1-Propenyltrimethylsilan 1,7%, Isopropenyltrimethylsilan 8,1% und Allyltrimethylsilan 15,3%.

Zur Isolierung des trans-1-Propenyllithiums (9) werden der Lösung 74,0 ml entnommen, im Vakuum (0,27 mbar) auf 33,0 ml eingeengt, mit 50 ml Pentan versetzt, 10 Min. gerührt und filtriert. Zur Kristallisation von (9) wir das Filtrat 3 Std. bei −40 °C und anschliessend 12 Std. bei −78 °C gehalten; die Kristalle von (9) werden bei −78 °C filtriert, dreimal mit je 40 ml kalten Pentans gewaschen, eine halbe Stunde bei −30 °C, eine halbe Stunde bei 0 °C und eine Stunde bei 20 °C im Vakuum (0,27 mbar) getrocknet. Man erhält 9,25 g des trans-1-Propenyllithium-Tetrahydrofuranadduktes in Form schwach braun gefärbter Kristalle (Li-Gehalt 6,51; Ausbeute 45,6% d. Th., bezogen auf Lithium). Das $^1H$-NMR-Spektrum des Produktes

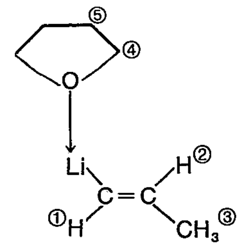

(9)

(80 MHz, 10% in $(C_2D_5)_2O$;
$\tau = 3,39$ d (H①), 3,78 m (H②), 6,17 m (H④), 8,10 m (H⑤), 8,18 d (H③); $J_{12} = 21$ Hz) stimmt mit dem von D. Seyferth und L.G. Vaughan (J. Organomet. Chem. 1, 201 [1963]) aus trans-1-Chlor-1-propen und Lithium dargestellten (9) überein.

Zur weiteren Reinigung werden 9,0 g des Rohproduktes aus einem Gemisch von 18 ml THF und 32 ml Pentan, wie oben beschrieben, umkristallisiert. Man erhält 6,5 g trans-1-Propenyllithium-Tetrahydrofuranaddukt in Form farbloser Kristalle. $C_3H_5Li \cdot C_4H_8O$ (MG = 120,0); ber. 5,78% Li; gef. 5,75 Li. Die Umsetzung von 6,0 g (49,7 mMol) dieses Produktes mit Trimethylchlorsilan, wie oben beschrieben, liefert 5,43 g $(CH_3)_3SiC_3H_5$ mit der Zusammensetzung: trans-1-Propenyltrimethylsilan 93,4%, cis-1-Propenyltrimethylsilan 0,4%, Isopropenyltrimethylsilan 1,3% und Allyltrimethylsilan 4,9%.

Beispiel 2

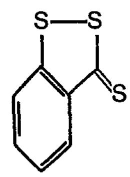

(3a)

Eine Lösung von 0,90 g (4,9 mMol) 4,5-Benzo-1,2-dithiol-3-thion (BDT) (3a) und 1,63 g (9,8 mMol) $FeCl_3$ in 100 ml THF wird bei 0 °C mit Propen (1 bar) gesättigt und anschliessend wird der Lösung in Propenatmosphäre bei 0 °C und unter Rühren 4,87 g (0,70 Mol) Lithiumsand zugegeben (Molverh. 3a:$FeCl_3$:Li = 1:2:143). Nach einem vorübergehenden Temperaturanstieg beginnt nach 10–15 Min. die Propenaufnahme. Während der Propenaufnahme wird die Suspension gerührt, der Propendruck bei 1,1–1,2 bar und die Temperatur bei 0 bis +2 °C gehalten. Das Reaktionsgemisch nimmt bis zur Sättigung innerhalb von 71 Std. 3,8 l Propen (1 bar, 20 °C) auf. Die Suspension wird filtriert und Lithiumhydrid mit THF gewaschen. 8,0 ml von insgesamt 114,0 ml des Filtrates werden, wie im Beispiel 1 beschrieben, hydrolysiert, wobei 351 ml Gas (1 bar, 20 °C) mit der Zusammensetzung Propen 75,7%, THF 7,8%, $H_2$ 8,7% und Acetylen 2,7% frei wird. Aus der Propenmenge errechnet sich nach Gl. 8 eine Ge-

samtausbeute an $LiC_3H_5$ von 45%. Bei der Silylierung eines aliquoten Teils des Filtrates, wie im Beispiel 1 beschrieben, erhält man ein Gemisch isomerer Silane $(CH_3)_3SiC_3H_5$ folgender Zusammensetzung: trans-1-Propenyltrimethylsilan 83,8%, cis-1-Propenyltrimethylsilan 1,3%, Isopropenyltrimethylsilan 10,3% und Allyltrimethylsilan 4,6%. Dieses Ergebnis bedeutet, dass im vorliegenden Fall die katalytische Lithiierung von Propen mit einer Selektivität von 83,8% in der trans-1-Stellung des Propens erfolgt.

Beispiele 3–12

Zur Darstellung des 2,4-Diphenyl-1,6,6a-trithiapentalen·$2CuCl_2$-Komplexes (15) (Beispiel 6) werden 3,09 g (23,0 mMol) wasserfreies

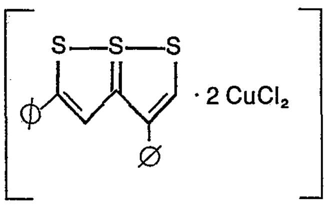
· 2 $CuCl_2$ 15

Kupfer(II)-chlorid in 100 ml Toluol suspendiert, mit 3,73 g (12,0 mMol) 2,4-Diphenyl-1,6,6a-trithiapentalen versetzt und die Mischung 18 Std bei Raumtemperatur gerührt. Die Suspension wird filtriert, der Niederschlag mit Toluol gewaschen und bei $1{,}33 \cdot 10^{-3}$ mbar getrocknet. Man erhält 4,0 g (60% d. Th.) des Komplexes (15). $C_{17}H_{12}S_3Cu_2Cl_4$ (580,8);

ber. C 35,12 H 2,07 S 16,56 Cu 21,88 Cl 24,41

gef. C 34,85 H 2,55 S 16,34 Cu 21,78 Cl 24,35.

Durchführung der Beispiele 3–12 (Tabelle 1): Die Katalysatorbestandteile werden in THF vorgelegt, die Suspension gegebenenfalls 12 Std. bei 20 °C gerührt, anschliessend werden die Ansätze bei der jeweiligen Reaktionstemperatur mit Propen (1 bar) gesättigt und Lithiumsand in Propenatmosphäre unter Rühren zugegeben. Die nach bestimmten Reaktionszeiten aufgenommenen Propenmengen (in l, 1 bar, 20 °C) sowie die Ausbeuten an $LiC_3H_5$ und die Isomerenverhältnisse (9:10:11:12) sind in der Tabelle 1 angegeben. Die Bestimmung der Ausbeuten und der Isomerenverhältnisse erfolgt, wie im Beispiel 1 beschrieben.

Tabelle 1: Katalytische Reaktion von Lithium mit $C_3H_6$ zu $LiC_3H_5$ und LiH

| Beispiel Nr. | Katalysator g [mMol] | THF [ml] | Li-Sand g [Mol] | Rkt.-temp. [°C] | Rkt.-zeit [Std.] | Propen-aufnahme [l] | $LiC_3H_5$ Ausbeute [%] | Zusammensetzung von $LiC_3H_5$ (9) % | (10) | (11) | (12)[a)] |
|---|---|---|---|---|---|---|---|---|---|---|---|
| 3 | 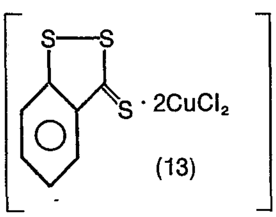 · $2CuCl_2$ (13) 1.31 (2.9) | 70 | 1.90 (0.27) | 0 | 74 | 2.54 | 75 | 62.4 | 1.8 | 14.9 | 20.9 |
| 4 | 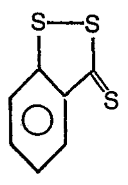 + $2CuCl_2$[b] 0.57 (4.2) 0.39 (2.1) | 50 | 1.30 (0.19) | −20 | 48 | 1.08 | 31 | 76.6 | 1.1 | 12.6 | 9.7 |
| 5 | 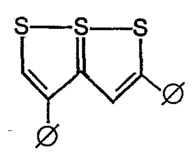 + $2CuCl_2$[b] 0.46 (3.4) 0.53 (1.7) | 70 | 1.60 (0.23) | 0 | 52 | 2.31 | 78 | 52.1 | 1.0 | 9.0 | 37.2 |
| 6 | 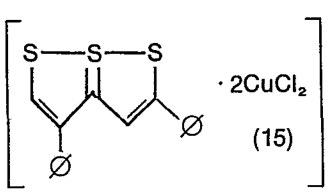 · $2CuCl_2$ (15) 1.01 (1.74) | 50 | 1.06 (0.15) | +20 | 48 | 0.79 | 43 | 58.2 | 1.1 | 0.4 | 40.2 |

| Bei-spiel Nr. | Katalysator g [mMol] | THF [ml] | Li-Sand g [Mol] | Rkt.-temp. [°C] | Rkt.-zeit [Std.] | Propen-aufnahme [l] | $LiC_3H_5$ Aus-beute [%] | Zusammensetzung von $LiC_3H_5$ (9) % | (10) | (11) | (12)[a] |
|---|---|---|---|---|---|---|---|---|---|---|---|
| 7 | 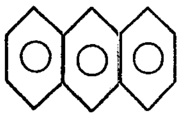 + $2CuCl_2$[b] 0.27 (1.5) 0.42 (3.1) | 50 | 1.42 (0.20) | 0 | 42 | 1.22 | 45 | 78.0 | 1.9 | 13.0 | 7.0 |
| 8 | 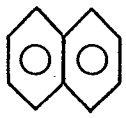 + $2CuCl_2$[c] 0.65 (5.1) 1.34 (10.0) | 150 | 4.80 (0.69) | 0 | 120 | d) | 41 | 79.2 | 1.6 | 8.9 | 10.3 |
| 9 | Ø—Ø + $2CuCl_2$[c] 0.83 (5.4) 1.46 (10.8) | 100 | 5.67 (0.82) | 0 | 67 | 2.58[d)] | 38 | | | | |
| 10 | 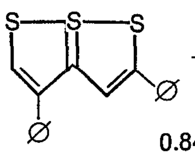 + $2FeCl_3$[b] 0.80 (2.6) 0.84 (5.2) | 70 | 1.70 (0.24) | 0 | 48 | 1.71 | 47 | 82.3 | 1.9 | 9.4 | 6.5 |
| 11 | 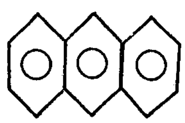 + $2FeCl_3$[c] 1.05 (5.9) 1.90 (11.7) | 100 | 3.96 (0.57) | 0 | 120 | 2.73[d)] | 26 | 82.5 | 1.7 | 9.7 | 6.2 |
| 12 | 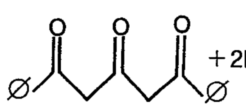 + $2FeCl_3$ 1.38 (5.2) 1.68 (10.4) | 100 | 5.27 (0.76) | 0 | 98 | 2.78[d)] | 22 | 85.5 | 2.0 | 10.2 | 2.3 |

a) (9): 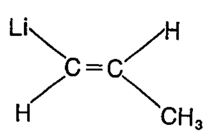 ; (10): 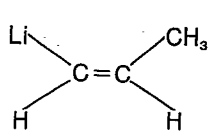 ; (11): 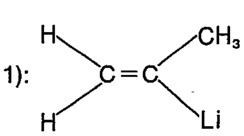 ; (12): 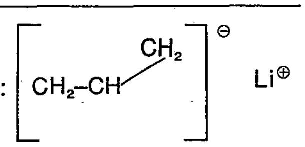

b) Vor der Zugabe von Li wurde die Probe jeweils 12 Std. bei 20°C gerührt.

c) Die jeweilige aromatische Verbindung und Metallsalz wurden in THF vorgelegt, die Lösung mit $C_3H_6$ gesättigt und Li zugegeben.

d) Es wurde bei einem Propendruck von 1.1–1.2 bar gearbeitet.

Beispiele 13–24

Durchführung der Beispiele 13–24 (Tabelle 2):

4,5-Benzo-1,2-dithiol-3-thion (BDT) (3a) und das jeweilige Metallsalz (Molverh. BDT:Metallsalz 1:2) werden in 60 ml THF 12 Std. bei 20 °C gerührt, anschliessend wird der Ansatz bei 0 °C mit Propen (1 bar) gesättigt und in Propenatmosphäre und unter Rühren Lithiumsand zugegeben. Die nach bestimmten Reaktionszeiten aufgenommenen Propenmengen (in l, 1 bar, 20 °C) sowie die Ausbeuten an $LiC_3H_5$ und die Isomerenverhältnisse (9:10:11:12) sind in der Tabelle 2 angegeben. Die

Bestimmung der Ausbeuten und der Isomerenverhältnisse erfolgt, wie im Beispiel 1 beschrieben.

Beispiele 25–27

Darstellung des ortho-Chlorpalladio-2,5-diphenyl-1,6,6a-trithiapentalen-Komplexes (8) (Beispiel 25):

Zu der Suspension von 3,10 g (9,94 mMol) 2,5-Diphenyl-1,6,6a-trithiapentalen in einem Gemisch von 230 ml Methanol und 25 ml Benzol gibt man 1,76 g (9,92 mMol) $PdCl_2$ und darauf 1,33 g (31,3 mMol)LiCl gelöst und 20 ml Methanol. Die Suspension wird 3 Std. unter Rühren am Rückfluss gekocht und nach Abkühlen auf Raumtemperatur durch einen G-3-Glasfiltertiegel filtriert. In der Mutterlauge wurden 93,6% des abgespaltenen HCl acidimetrisch bestimmt. Der Niederschlag wird mit Methanol und Äther gewaschen und bei $1{,}33 \cdot 10^{-3}$ mbar getrocknet; Die Ausbeute an (8) (Schmp. 304 °C, Zers.) beträgt 4,38 g (97%).

$C_{17}H_{11}S_3PdCl$ (453,8);

ber. C 44,94 H 2,64 S 21,15 Pd 23,44 Cl 7,82

gef. C 44,92 H 2,90 S 21,08 Pd 23,21 Cl 7,86.

Durchführung der Beispiele 25–27 (Tabelle 3):

Tabelle 2: Katalytische Reaktion von Lithium mit $C_3H_6$ zu $LiC_3H_5$ und LiH

(bei 0°C und 1 bar Propen in 60 ml THF; Molverh. 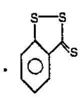 (BDT):$MeX_n$ = 1:2)

| Bei-spiel Nr. | Katalysator g (mMol) | Li-Sand g (Mol) | Rkt.-zeit [Std.] | Propen-aufnahme [l] | $LiC_3H_5$-Ausbeute [%] | Zusammensetzung von $LiC_3H_5$ (9) [%] | (10) | (11) | (12)[a)] |
|---|---|---|---|---|---|---|---|---|---|
| 13 | BDT+2$TiCl_4$ 0.33 (1.8) 0.68 (3.6) | 1.14 (0.16) | 46 | 1.00 | 29 | 84.8 | 2.0 | 6.5 | 6.7 |
| 14 | BDT+2$CrCl_3$ 0.31 (1.7) 0.56 (3.5) | 1.20 (0.17) | 46 | 1.10 | 42 | 83.1 | 1.9 | 5.5 | 9.4 |
| 15 | BDT+2$MoCl_5$ 0.33 (1.8) 0.99 (3.6) | 1.08 (0.16) | 53 | 0.78 | 31 | 68.0 | 1.6 | 20.0 | 10.4 |
| 16 | BDT+2$MnCl_2$ 0.44 (2.4) 0.60 (4.8) | 1.33 (0.19) | 45 | 1.38 | 37 | 75.7 | 2.4 | 7.0 | 15.0 |
| 17 | BDT+2$CoCl_2$ 0.37 (2.0) 0.53 (4.1) | 1.07 (0.15) | 29 | 1.33 | 52 | 80.7 | 2.0 | 8.2 | 9.1 |
| 18 | BDT+2$NiCl_2$ 0.32 (1.7) 0.44 (3.4) | 1.30 (0.19) | 72 | 1.22 | 40 | 64.8 | 1.7 | 2.4 | 31.1 |
| 19 | BDT+2$Niacac_2$[b)] 0.29 (1.6) 0.80 (3.2) | 0.99 (0.14) | 30 | 0.63 | | 67.0 | 2.3 | 2.3 | 28.4 |
| 20 | BDT+2$ZnCl_2$ 0.41 (2.2) 0.61 (4.5) | 1.68 (0.24) | 48 | 1.60 | 53 | 83.1 | 1.0 | 9.9 | 6.0 |
| 21 | BDT+2$RhCl_3$. 0.22 (1.2) 0.49 (2.3) | 0.76 (0.11) | 48 | 0.43 | 39 | 20.9 | 1.2 | 6.7 | 71.2 |
| 22 | BDT+2$PtCl_2$ 0.27 (1.5) 0.77 (2.9) | 1.05 (0.15) | 48 | 1.00 | 51 | 28.0 | 0.3 | 3.4 | 68.2 |
| 23 | 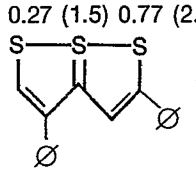 +2$PtCl_2$ 0.29 (0.9) 0.49 (1.8) | 0.88 (0.13) | 29 | 1.01 | 66 | 39.6 | 0.6 | 5.9 | 53.8 |
| 24 | BDT+2$PdCl_2$ 0.30 (1.6) 0.57 (3.2) | 1.10 (0.16) | 72 | 1.00 | 54 | 55.8 | 1.0 | 7.6 | 35.6 |

a) (9): 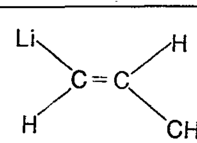 ; (10): 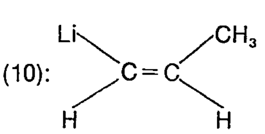 ; (11): 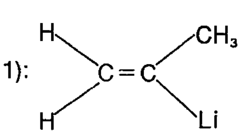 ; (12): 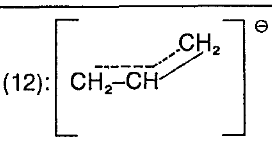 $Li^{\oplus}$

b) $Niacac_2$ = Nickelacetylacetonat

Die Lösung bzw. die Suspension des Katalysators in THF wird bei 0 °C mit Propen (1 bar) gesättigt und anschliessend wird in Propenatmosphäre und unter Rühren Lithiumsand zugegeben. Die nach bestimmten Zeiten aufgenommenen Propenmengen (in l, 1 bar, 20 °C) sowie die Ausbeuten an $LiC_3H_5$ und die Isomerenverhältnisse (9:10:11:12) sind in der Tabelle 3 angegeben. Die Bestimmung der Ausbeuten und der Isomerenverhältnisse erfolgt, wie im Beispiel 1 beschrieben.

Tabelle 3: Katalytische Reaktion von Lithium mit Propen zu $LiC_3H_5$ und LiH (bei 0°C)

| Beispiel Nr. | Katalysator g (mMol) | Lsg. mittel (ml) | Li-Sand g (Mol) | Rkt. zeit [Std.] | Propen-aufnahme [l] | $LiC_3H_5$-Ausbeute [%] | Zusammensetzung von $LiC_3H_5$ (9) [%] | (10) | (11) | (12)[a] |
|---|---|---|---|---|---|---|---|---|---|---|
| 25 | 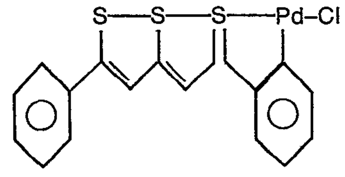 (8) 1.69 (3.7) | THF (50) | 2.45 (0.35) | 95 | 2.58 | 59 | 10.2 | 0.2 | 0.6 | 89.1 |
| 26 | BDT[b] 0.58 (3.2) | THF (100) | 5.09 (0.73) | | 2.48[c] | 22 | 77.4 | 1.7 | 13.0 | 8.0 |
| 27 | 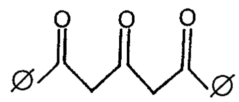 1.89 (7.2) | THF (50) | 1.53 (0.22) | | 1.30 | 36 | | | | |

a) (9): 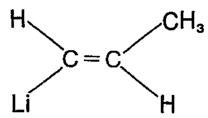 ; (10): 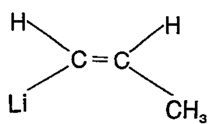 ; (11): 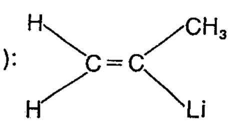 ; (12): 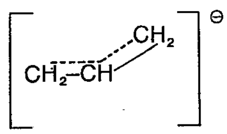 $Li^{\oplus}$

b) BDT = 4,5-Benzo-1,2-dithiol-3-thion

c) Es wurde bei einem Propendruck von 1.1–1.2 bar gearbeitet.

Beispiele 28–33

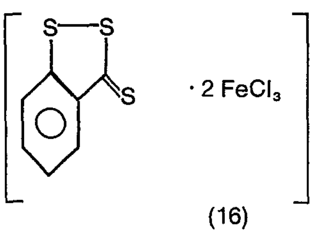

$\cdot 2\ FeCl_3$

(16)

Darstellung des 4,5-Benzo-1,2-dithiol-3-thion·$2FeCl_3$-Komplexes (16) (Beispiel 29):

Zu der Suspension von 1,89 g (11,6 mMol) des wasserfreien $FeCl_3$ in 80 ml Benzol wird unter Rühren die Lösung von 1.07 g (5,8 mMol) 4,5-Benzo-1,2-dithiol-3-thion (3a) in 70 ml Benzol zugetropft, anschliessend wird die Mischung 24 Std. bei 20 °C gerührt. Die Suspension wird filtriert, der Niederschlag mit Benzol gewaschen und bei $1,33 \times 10^{-3}$ mbar getrocknet. Man erhält 2,36 g (80% d. Th.) des Komplexes (16). $C_7H_4S_3Fe_2Cl_6$ (508,7);

ber. C 16,52 H 0,78 Fe 21,97 S 18,90 Cl 41,84
gef. C 16,55 H 0,82 Fe 21,91 S 18,84 Cl 41,76.

Durchführung der Beispiele 28–33 (Tabelle 4):

Die Katalysatoren werden in THF vorgelegt, die Lösung bei 0 °C mit Äthylen (1 bar) gesättigt und anschliessend wird bei 0 °C und unter Rühren in Äthylenatmosphäre Lithiumsand zugegeben. Die nach bestimmten Reaktionszeiten aufgenommenen Äthylenmengen (in l, 1 bar und 20 °C) sind in der Tabelle 4 angegeben. Die Suspensionen werden filtriert und Lithiumhydrid wird mit THF gewaschen. Zur Bestimmung der Ausbeute an Vinyllithium im Filtrat werden aliquote Teile der Filtrate im Vakuum eingedampft und die Rückstände hydrolisiert. Aus den entwickelten Äthylenmengen und aufgrund der Gl. 7 werden die in der Tabelle 4 angegebenen Ausbeuten an Vinyllithium errechnet.

Zur Isolierung des Vinyllithiums werden im Beispiel 28 85 ml von insgesamt 90 ml des Filtrates im Vakuum (0,27 mbar) eingedampft, der Rückstand mit 50 ml Pentan 30 Min. gerührt, die Suspension filtriert und der Festkörper viermal mit je 10 ml Pentan gewaschen. Beim Kühlen des Filtrates auf −40 °C kristallisiert Vinyllithium-Tetrahydrofuranaddukt (2,72 g) in Form farbloser Kristalle. $C_6H_{11}OLi$ (106,1);

ber. 6,60% Li;
gef. 6,60% Li.

Zur Bestimmung des Lithiumhydrids wird im Beispiel 32 das bei der Filtration gewonnene Lithiumhydrid bei 0,27 mbar getrocknet, wobei man 4,1 g eines grauen Pulvers mit 46,7% Li erhält. 0,158 g dieses Pulvers liefern bei der Zersetzung: HD 7,50%, $D_2$ 6,3%, $H_2$ 6,3%, $C_2H_3D$ 2,1% und THF 1,3%. Aus der Menge von HD errechnet sich nach Gl. 7 eine Ausbeute an LiH von 69%.

Tabelle 4: Katalytische Reaktion von Lithium mit Äthylen zu Vinyllithium und Lithiumhydrid (bei 0°C)

| Beispiel Nr. | Katalysator g (mMol) | Lsg. mittel (ml) | Li-Sand g (Mol) | Rkt.-zeit [Std.] | Äthylen-aufnahme [1] | $LiC_2H_3$-Ausbeute [%] |
|---|---|---|---|---|---|---|
| 28 | 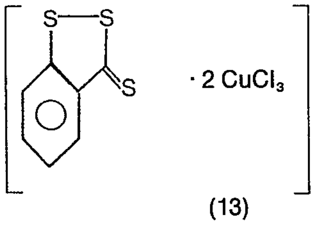 · 2 $CuCl_3$ (13) 1.07(2.4) | THF (70) | 1.70 (0.24) | 44 | 2.42 | 60 |
| 29 | 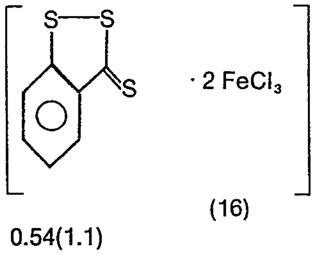 · 2 $FeCl_3$ (16) 0.54(1.1) | THF (50) | 0.55 (0.08) | 24 | 0.68 | 72 |
| 30 | 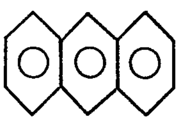 + 2$FeCl_3$[a] 2.1(1.2)3.9(2.4) | THF (50) | 0.82 (0.12) | 70 | 0.93 | 64 |
| 31 | 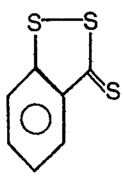 0.90(4.9) | THF (50) | 1.91 (0.28) | 143 | 2.03[b)] | 35 |
| 32 | 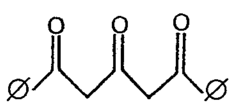 1.13(4.3) | THF (100) | 3.62 (0.52) | 130 | 5.00[b)] | 44 |
| 33 | 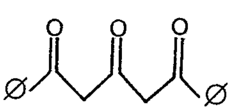 + 2$FeCl_3$ 0.96(3.6) 1.1(6.8) | 1,2–Dimeth-oxyäthan (100) | 1.94 (0.28) | 67 | 0.83[b)] | |

a) Vor der Zugabe von Li wurde die Probe 12 Std. bei 20°C gerührt.
b) Es wurde bei einem Äthylendruck von 1.1–1.3 bar gearbeitet.

Beispiel 34

In 100 ml abs. THF werden nacheinander 0,78 g (4,2 mMol) 4,5-Benzo-1,2-dithiol-3-thion (3a) 1,38 g (8,5 mMol) des wasserfreien $FeCl_3$ und bei 0 °C 24,2 g (0,43 Mol) 1-Buten gelöst und anschliessend wird die Lösung bei 0 °C und unter Rühren mit 7,30 g (1,05 Mol) Lithiumsand versetzt. Das Reaktionsgemisch wird insgesamt 7 Tage bei 0 °C gerührt. Während dieser Zeit werden der Lösung jeweils 5,0 ml Proben entnommen, filtriert und ihr Lithiumgehalt acidimetrisch bestimmt. Nach 17 bzw. 70 Std. Reaktionszeit findet man in den Proben 6,75 bzw. 10,6 g Atom Lithium, entsprechend einem Lithiumumsatz zu Lithiumbutenyl und Lithiumhydrid gemäss Gleichung 9 von 26 bzw. 40%. Nach 7 Tagen Reaktionszeit wird das Reaktionsgemisch bei 0 °C von Lithiumhydrid und nicht umgesetztem Lithium abfiltriert und der Niederschlag mit THF gewaschen. Eine 4,5 ml Probe des Filtrates (von insgesamt 138 ml) liefert bei der Hydrolyse 218 ml Gas (20 °C, 1 bar) mit 62,5 Vol.-% Buten-1; daraus errechnet sich nach Gl. 9 eine Ausbeute an $LiC_4H_7$ von 40%.

Zur Charakterisierung des Lithiumbutenyls wird die restliche Menge mit überschüssigem Trimethylchlorsilan in Äther, wie im Beispiel 1 beschrieben, umgesetzt. Man erhält 26,2 g eines Gemisches der vier isomeren Verbindungen $(CH_3)_3SiC_4H_7$ (Sdp. 95–109 °C/1,10 bar), von denen die Hauptkomponente laut Gaschromatogramm zu 87,9% vertreten ist. Nach dem $^1$H-NMR-Spektrum handelt es sich bei der Hauptkomponente um

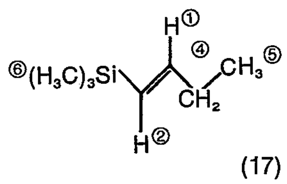

(17)

(100 MHz, 15% in $C_6H_6$, $\tau = 3{,}89$ m ($H^{①}$), 4,34 d ($H^{②}$), 7,98 m ($H^{④}$), 9,08 t ($H^{⑤}$) und 9,91 s ($H^{⑥}$); $J_{12} = 18{,}5$ Hz) das trans-1-Trimethylsilyl-1-buten (17), was bedeutet, dass die Lithiierung mit einer Selektivität von 87,9% in der 1-trans-Stellung von 1-Buten erfolgt.

Beispiel 35

Analog dem Beispiel 34 lässt man 41,4 g (0,74 Mol) 1-Buten in Gegenwart von 2,15 g (4,75 mMol) des Komplexes 13 (Beispiel 1) als Katalysator mit 5,60 g (0,81 Mol) Lithiumsand in 150 ml THF 10 Tage bei 0 °C reagieren. Das Gemisch wird filtriert und der Feststoff (LiH + Li) mit THF gewaschen. 5,0 ml des Filtrates (von insgesamt 186 ml) liefern nach Abdampfen des THF und anschliessender Hydrolyse 191 ml Gas (20 °C, 1 bar) mit 80% 1-Buten (Rest: THF, $H_2$ und $C_2H_2$); daraus errechnet sich nach Gl. 9 eine Ausbeute an $LiC_4H_7$ von 58% (bez. auf Li). Zur Isolierung des trans-1-Lithio-1-butens wird von dem restlichen Filtrat 110 ml THF im Vakuum (0,27 mbar) abdestilliert, 100 ml Pentan zugegeben und das Gemisch bei 0 °C von Katalysatorresten filtriert. Beim Stehen des Filtrats bei –78 °C über Nacht werden weitere Reste des Katalysators ausgeschieden. Die überstehende Lösung wird im Vakuum (0,27 mbar) vollständig eingedampft, der Rückstand mehrere Std. bei $1{,}33 \times 10^{-3}$ mbar getrocknet, in 120 ml Pentan aufgenommen, kurzzeitig gerührt und filtriert. Der weisse Frittenrückstand wird mit Pentan gewaschen und bei 0,2 Torr getrocknet. Man erhält 7,8 g trans-1-Lithium-1-buten mit einem Anteil von 9,48% Li. Nach der Sylilierung dieses Produktes mit Trimethylchlorsilan, Aufarbeitung und Destillation, wie im Beispiel 1 beschrieben, gewinnt man 97%iges trans-1-Trimethylsilyl-1-buten (nach GC-Analyse), das $^1$H-NMR-spektroskopisch identifiziert wird.

Beispiel 36

In einer Suspension von 0,58 g (1,28 mMol) 8 (s. Beispiele 25–27) in 20 ml THF wird 1,81 l (76 mMol) gasförmiges 1-Buten gelöst und anschliessend wird die Suspension bei 0° unter Rühren mit 0,92 g (0,13 Mol) Lithiumsand versetzt. Nach 50stündigem Rühren bei 0° wird filtriert und Lithiumhydrid mit THF gewaschen. Ein aliquoter Teil der Lösung (4,0 ml von insgesamt 43,6 ml) liefert nach Abdampfen des THF und Hydrolyse 200 ml Gas (20 °C, 1 bar) mit insgesamt 30,0% Butenen. Aus der Butenmenge errechnet sich eine Ausbeute an $LiC_4H_7$ von 40,7%. Bei der Umsetzung eines aliquoten Teils der Lösung mit Trimethylchlorsilan, wie im Beispiel 1 beschrieben, erhält man ein Gemisch der isomeren Silane $(H_3C)_3SiC_4H_7$, bei dem es sich nach $^1$H-NMR-Spektrum bzw. GC-Analyse überwiegend um ein Gemisch aus cis- und trans-1-Trimethylsilyl-2-buten handelt.

Beispiel 37

Einer Suspension von 6,29 g (0,91 Mol) Lithiumsand in 100 ml THF werden bei 0 °C und unter Rühren nacheinander 33,8 g (0,48 Mol) 1-Penten und 1,45 g (2,85 mMol) des Komplexes 16 (Beispiele 28–33) zugegeben. Das Gemisch wird 5 Tage bei 0 °C gerührt, anschliessend wird filtriert und der Feststoff (LiH) mit THF gewaschen. 2,50 ml des Filtrates (von insgesamt 169 ml) enthalten nach der acidimetrischen Lithiumbestimmung 4,30 gAtom Li, entsprechend einer Ausbeute an $LiC_5H_9$ von 64%.

Zur Charakterisierung der Organolithiumverbindung $LiCH_9$ werden 86,5 ml des Filtrates, wie im Beispiel 1 beschrieben, mit überschüssigem Trimethylchlorsilan umgesetzt. Die Aufarbeitung bzw. Destillation liefert u.a. 10,6 g einer Fraktion (Sdp. 133 °C/1,01 bar), bei der sich nach $^1$H-NMR-Spektrum (80 MHz, 15% in $CDCl_3$; $\tau$ 3 = 0,96 m

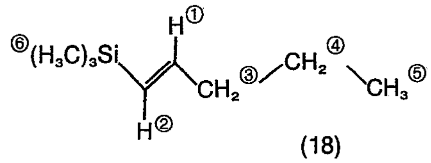

(18)

($H^{①}$), 4,39 d ($H^{②}$), 7,90 m ($H^{③}$), 8,57 m ($H^{④}$), 9,09 t ($H^{⑤}$), 9,94 s ($H^{⑥}$), $J_{12} = 18{,}5$ Hz) um das trans-1-Trimethylsilyl-1-penten (18) handelt.

Zur Isolierung des trans-1-Lithio-1-penten werden 80 ml des Filtrates im Vakuum auf 20 ml eingeengt, mit 80 ml Pentan versetzt und filtriert. Das Filtrat wird 12 Std. bei −78 °C gehalten und danach bei −78 °C von den ausgeschiedenen Katalysatorresten abgehebert. Die erhaltene Lösung wird im Vakuum vollständig eingedampft, der Rückstand bei 20 °C und $1{,}33 \times 10^{-3}$ bar bis zur Gewichtskonstanz getrocknet, in 100 ml Pentan aufgenommen, eine halbe Stunde gerührt und filtriert. Der weisse Niederschlag wird mit Pentan gewaschen und bei 0,27 mbar getrocknet. Man erhält 3,14 g des trans-1-Lithio-1-penten in Form eines weissen Pulvers. $LiC_5H_9$ (MG = 75,9);

ber. 9,32% Li;

gef. 9,29% Li.

Beispiel 38

Analog dem Beispiel 34 lässt man 21,6 g (0,20 Mol) 1,7-Octadien in Gegenwart von 1,25 g (2,76 mMol) des Komplexes 13 (Beispiel 1) als Katalysator mit 5,82 g (0,84 Mol) Lithiumsand in 150 ml THF 11 Tage bei 0 °C reagieren. Die Suspension wird filtriert und Lithiumhydrid mit THF gewaschen. 5,0 ml des Filtrates (von insgesamt 172 ml) enthalten nach der acidimetrischen Bestimmung 6,94 g Atom Lithium, entsprechend einer Gesamtausbeute an Organolithiumverbindungen von 57%.

Die in Lösung befindlichen Organolithiumverbindungen werden in Form ihrer Trimethylsilylderivate charakterisiert. Dazu werden 77 ml (von insgesamt 172 ml) der Lösung, wie im Beispiel 1 beschrieben mit Trimethylchlorsilan umgesetzt. Die Aufarbeitung bzw. Destillation liefert 7,67 g einer Fraktion Sdp. 55–63 °C/0,93 mbar sowie 1,55 g einer Fraktion Sdp. 54–55 °C/ 1,33 × $10^{-3}$ mbar. Nach dem $^1$H-NMR-Spektren besteht die erste Fraktion aus trans-1-Trimethylsilyl-1,7-octadien (19) und die zweite Fraktion im wesentlichen aus Bis-1,8-(trans)-trimethylsilyl-1,7-octadien (20), d.h.

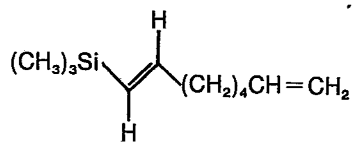

(19)

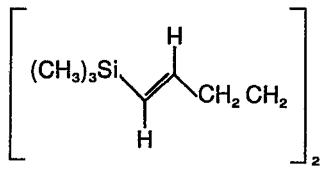

(20)

dass auch im Falle des 1,7-Otadiens die Lithiierung mit einer hohen Selektivität in der trans-1-Stellung erfolgt.

Beispiel 39

In 150 ml abs. THF werden nacheinander 1,79 g (3,95 mMol) des Komplexes 13 (Beispiel 1), sowie 13,0 g (0,19 Mol) 1,4-Pentadien suspendiert bzw. gelöst und anschliessend der Suspension bei 0 °C unter Rühren 11,1 g (1,60 Mol) Lithiumsand zugegeben. Das Reaktionsgemisch wird 74 Std. bei 0 °C gerührt. Nach dieser Zeit enthält eine 5,0-ml-Probe der Lösung 9,41 mg Atom Lithium. Die Suspension wird mit 50 ml THF verdünnt, bei 0 °C filtriert und LiH mit THF gewaschen. Während des 48stündigen Stehens der Lösung bei −78 °C kristallisierte die Organolithiumverbindung 14 in Form braungefärbter, derber Kristalle aus. Die Kristalle werden bei −78 °C von der Mutterlauge getrennt, mit wenig −78 °C kaltem THF gewaschen, eine halbe Stunde bei 0 °C und eine halbe Stunde bei 20 °C im Vakuum (0,27 mbar) getrocknet. Man erhält 11,1 g Produkt mit einem Anteil von 9,61% Lithium (ber. für $C_4H_5Li_3 \cdot (THF)_2$ 9,0% Li). Aufgrund der $^1$H- bzw. $^{13}$C-NMR-Spektren in Kombination mit Spin-Spin-Entkopplungsexperimenten, sowie der Silylierung (s. unten), wird der Organolithiumverbindung die Struktur 14 zugeordnet. Für die Aufnahme des $^1$H-NMR-Spektrums wird das Produkt mit 9,61% Li zweimal aus einem 1:1-THF/Tetramethyläthylendiamin-Gemisch umkristallisiert (Kristallisation jeweils bei −78 °C). Das $^1$H-NMR-Spektrum von 14 (15%ig in $d_8$-THF; 270 MHz; 27 °C; d-THF als interner Standard): δ = 7,54 dd (H①), 5,37 d (H③), 4,79 d (H④), 3,03 d (H⑤), 2,95 d (②), 3,54 m und 1,68 m (THF), 2,21 s und 2,06 s (Tetramethylendiamin); $J_{12}$ = 16,3 Hz, $J_{13}$ = 5,4 Hz, $J_{45}$ = 4,2 Hz.

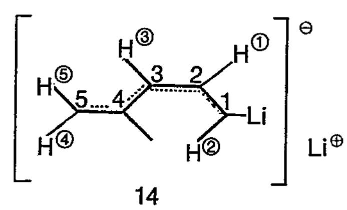

14

Für die Aufnahme des $^{13}$C-NMR-Spektrums wird das Rohprodukt aus THF umkristallisiert (Kristallisation bei −78 °C). Das $^{13}$C-NMR-Spektrum von 14 (100 MHz; 10%ig in $d_8$-THF, bei 25 °C): δ (ppm) = 84,6 t ($C^5$), 97,6 (breit) ($C^1$), 100,4 d ($C^3$), 153,9 d ($C^2$), 187,7 (breit) ($C^4$). Die Verbreiterung der Signale der $^{13}C^1$- und $^{13}C^4$-Kerne zeigt das Vorhandensein zweier Li-C-Bindungen an. Bei der Reaktion von 1,07 g 14 mit Trimethylchlorsilan, wie im Beispiel 1 beschrieben, erhält man nach Aufarbeitung bzw. Destillation 0,85 g einer Fraktion Sdp. 45–47 °C/1,33 × $10^{-4}$ mbar, bei der es sich gemäss dem $^1$H-NMR-Spektrum um ein Gemisch der drei stereoisomeren 1,4,5-Tris(trimethylsilyl)-1,3-pentadiene (21) (65%), (22) (33%) und (23) (2%) handelt.

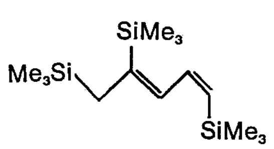

21

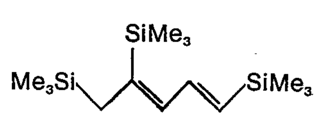

22

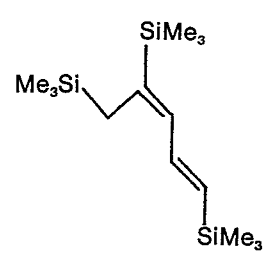

23

In einem Parallelversuch wurde gezeigt, dass bei der Reaktion von Pentadien-1,4 mit Lithium unter den gleichen Reaktionsbedingungen, jedoch in Abwesenheit des Katalysators, die Bildung von 14 höchstens in Spuren erfolgt.

Beispiel 40

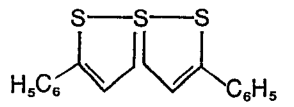

24

Eine Lösung von 0,34 g (1,1 mMol) 2,5-Diphenyl-1,6,6a-trithiapentalen 24 und 0,30 g (2,2 mMol) $ZnCl_2$ (wasserfrei) in 50 ml abs. THF wird bei 0 °C mit Ethylen (1 bar) gesättigt und anschliessend wird der Ansatz in Ethylenatmosphäre bei 0 °C und unter Rühren mit 1,45 g (0,21 Mol) Lithiumsand versetzt. Nach einem leichten Temperaturanstieg beginnt nach 10–15 Minuten die Ethylenaufnahme, deren Geschwindigkeit mit Hilfe einer an das Reaktionsgefäss angeschlossenen Gasbürette gemessen wird. Während der Ethylenaufnahme wird die Suspension kräftig geführt und die Temperatur bei 0 °C gehalten. Bis zur Beendigung der Reaktion nimmt das Reaktionsgemisch innerhalb von 6 Stunden 2,28 l Ethylen (1 bar, 20 °C) auf. Die Suspension wird von Lithiumhydrid filtriert und Lithiumhydrid mit THF gewaschen. 50 ml von insgesamt 81,0 ml des Filtrats liefern nach Abdampfen des THF bei der Hydrolyse 126 ml Gas (20 °C, 1 bar), das nach der MS-Analyse zu 84,8 Mol% aus Ethylen besteht. Aus der bei der Hydrolyse erhaltenen Ethylenmenge errechnet sich nach der Gleichung 7 eine Vinyllithiumausbeute von 76% (bez. auf Ethylen).

Beispiel 41

Eine Lösung von 1,61 g (5,2 mMol) 2,5-Diphenyl-1,6,61-trithiapentalen (24) und 1,21 g (8,9 mMol) $ZnCl_2$ (wasserfrei) in 100 ml abs. THF wird bei 0 °C mit Propen (1 bar) gesättigt und anschliessend wird der Ansatz in Propenatmosphäre bei 0 °C und unter Rühren mit 5,47 g (0,79 Mol) Lithiumsand versetzt. Die weitere Durchführung des Versuches erfolgt wie im Beispiel 40 für Ethylen beschrieben. Bis zur Beendigung der Reaktion nimmt das Reaktionsgemisch innerhalb von 12 Stunden 7,9 l Propen (20 °C, 1 bar) auf. Die Suspension wird filtriert und Lithiumhydrid mit THF gewaschen. 7,0 ml von insgesamt 167,0 ml des Filtrates liefern bei der Hydrolyse 372 ml Gas (20 °C, 1 bar), das zu 88,8 Mol% aus Propen besteht (Rest: THF, $H_2$). Aus der bei der Hydrolyse enthaltenen Propenmenge errechnet sich nach Gleichung 8 eine Ausbeute an Organolithiumverbindungen $LiC_3H_5$ von 99,7% (bezogen auf Propen). Die Umsetzung von 40 ml des Filtrates mit Trimethylchlorsilan und die anschliessende Aufarbeitung und Destillation, wie in Beispiel 1 beschrieben, liefern 11,9 g der isomeren Silane $(CH_3)_3SiC_3H_5$ der Zusammensetzung: trans-1-Propenyltrimethylsilan 80,0%, cis-1-Propenyl-trimethylsilan 0,4%, Isopropenyltrimethylsilan 15,0% und Allyltrimethylsilan 4,6%. Die Isolierung der Organolithiumverbindungen $LiC_3H_5$ aus der THF-Lösung, wie in Beispiel 1 beschrieben, liefert ein Produkt, das zu 91,3% aus trans-Propenyl-lithium besteht.

Beispiel 42

Zu einer Lösung von 25,1 g (0,22 Mol) 1-Okten und 1,23 g (4,0 mMol) 2,5-Diphenyl-1,6,6a-trithiapentalen (24) in 100 ml abs. THF werden bei 0 °C und unter Rühren nacheinander 1,15 g (8,5 mMol) $ZnCl_2$ (wasserfrei) und in kleinen Portionen 3,09 g (0,45 Mol) Lithiumsand gegeben. Der Ansatz wird insgesamt 22 Stunden bei 0 °C gerührt. Während dieser Zeit werden der Lösung jeweils 2,5 ml Proben entnommen, filtriert und der Lithiumgehalt in den Filtraten acidimetrisch bestimmt. Nach 3,5, 6 bzw. 22 Stunden beträgt der Lithiumgehalt in den Proben 4,2, 4,6 bzw. 5,4 mMol, entsprechend einem Lithiumsatz zu Lithiumoktenyl und Lithiumhydrid gemäss Gleichung 9 von 75, 83 bzw. 97%. Der Ansatz wird filtriert und Lithiumhydrid mit THF gewaschen. 47,0 von insgesamt 167,0 ml des Filtrats werden, wie im Beispiel 1 beschrieben, mit 11,0 g (0,10 Mol) Trimethylchlorsilan umgesetzt. Die Aufarbeitung bzw. Destillation liefert 6,13 g einer Fraktion Sdp. 35–43 °C/0,27 mbar, die nach der GC-Analyse bzw. GC-MS-Kopplungsanalyse und $^1H$-NMR-Spektrum zu 96,6% aus dem trans-1-Trimethyl-silyl-1-ikten (25) besteht. Nach diesem

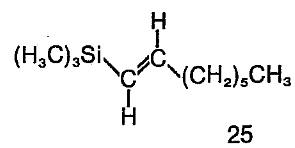

25

Ergebnis wird 1-Okten nach der beschriebenen Methode mit einer Selektivität > 96% in der trans-1-Stellung lithiert.

**Patentansprüche**

1. Verfahren zur Herstellung von Organolithiumverbindungen neben Lithiumhydrid, dadurch gekennzeichnet, dass man Lithium in Gegenwart eines Katalysators aus einer Mischung von Heteroverbindungen I–V,

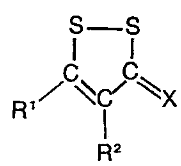 I

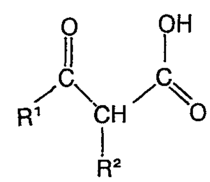 II

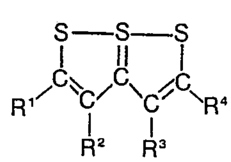 III

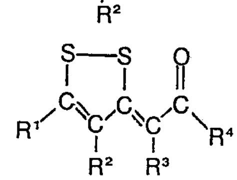 IV

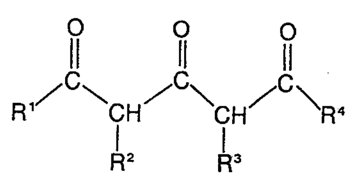 V

in denen X Schwefel oder Sauerstoff, $R^1$, $R^2$, $R^3$ und $R^4$ Wasserstoff, Alkyl-, Cycloalkyl-, Aralkyl- oder Arylreste bedeuten und/oder 2 oder mehrere solcher Reste zu einem aliphatischen oder aromatischen Ringsystem geschlossen sind, mit einer Übergangsmetallverbindung von Metallen der I., II., IV.–VI. und VIII. Nebengruppe des Periodensystems oder polycyclischen Aromaten wie Anthracen, Naphthalin und Biphenyl mit einer Übergangsmetallverbindung von Metallen der I., II., IV.–VII. und VIII. Nebengruppe des Periodensystems in einem Lösungsmittel mit einem α-Olefin oder α,ω-Diolefin umsetzt.

2. Verfahren nach Anspruch 1, dadurch gekennzeichnet, dass man Addukte zwischen den genannten Verbindungen III–VII und den genannten Übergangsmetallverbindungen, insbesondere Kupfer-, Eisen- und Palladiumhalogenide isoliert und anschliessend auf Lithium und Olefine bzw. Diolefine einwirken lässt.

3. Verfahren nach Anspruch 2, dadurch gekennzeichnet, dass ein Reaktionsprodukt der Formel

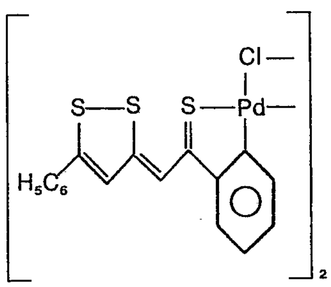

oder

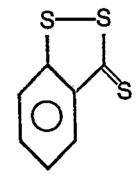

$\cdot 2CuCl_2$ oder

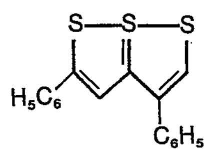

$\cdot 2CuCl_2$

oder

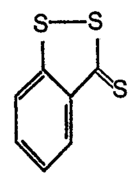

$\cdot$ $2FeCl_3$

mit Lithium umgesetzt wird.

4. Verfahren nach Ansprüchen 1–3, dadurch gekennzeichnet, dass die Metalle Kupfer, Gold, Zink, Cadmium, Titan, Zirkon, Vanadin, Niob, Tantal, Chrom, Molybdän, Wolfram, Mangan, Eisen, Kobalt, Nickel, Rhutenium, Rhodium, Palladium, Osmium, Iridium und Platin sind.

5. Verfahren nach Ansprüchen 1–4, dadurch gekennzeichnet, dass die Metalle Kupfer, Eisen, Zink, Palladium, Platin und Rhodium sind.

6. Verfahren nach Ansprüchen 1–5, dadurch gekennzeichnet, dass die Umsetzung in einem cyclischen oder einem offenkettigen Mono- oder Polyäther als Lösungsmittel, bevorzugt in Tetrahydrofuran, durchgeführt wird.

7. Verfahren nach Ansprüchen 1–6, dadurch gekennzeichnet, dass bei Temperaturen zwischen −100 bis +100 °C, bevorzugt zwischen −20 und +50 °C gearbeitet wird.

8. Verfahren nach Ansprüchen 1–7, dadurch gekennzeichnet, dass bei Partialdrucken unter 1 bis 100 bar gearbeitet wird.

9. Verfahren nach Ansprüchen 1–8, dadurch gekennzeichnet, dass α-Olefine der allgemeinen Formel $CH_2=CHR$, in der R = H, Alkyl, Aryl, Cycloalkyl und Aralkyl bedeutet, oder α,ω-Diolefine der allgemeinen Formel $CH_2=CH-(CHR)_n-CH=CH_2$, in der R die obige Bedeutung hat und n = 1 bis 6 ist, zum Einsatz kommen.

10. Addukte zwischen Heteroverbindungen der allgemeinen Formeln III bis VII

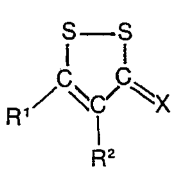

I

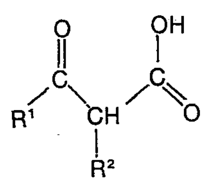

II

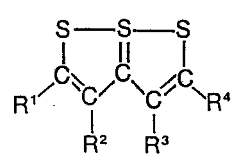

III

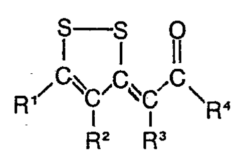

IV

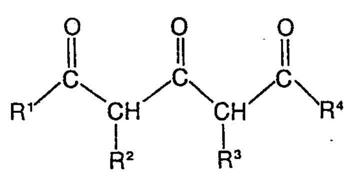

V

in denen X Schwefel oder Sauerstoff, $R^1$, $R^2$, $R^3$ und $R^4$ Wasserstoff, Alkyl-, Cycloalkyl-, Aralkyl- oder Arylreste bedeuten und/oder 2 oder mehrere solcher Reste zu einem aliphatischen oder aromatischen Ringsystem geschlossen sind, und einer Übergangsmetallverbindung von Metallen der I., II., IV.–VII. und VIII. Nebengruppe des Periodensystems.

11. Addukte nach Anspruch 10, dadurch gekennzeichnet, dass die Metalle Kupfer, Gold, Zink,

Cadmium, Titan, Zirkon, Vanadin, Niob, Tantal, Chrom, Molybdän, Wolfram, Mangan, Eisen, Kobalt, Nickel, Ruthenium, Rhodium, Palladium, Osmium, Iridium und Platin sind.

12. Addukte nach Anspruch 10, dadurch gekennzeichnet, dass die Metalle Kupfer, Eisen, Zink, Palladium, Platin und Rhodium sind.

13. Addukte nach Ansprüchen 10–12 und nach Anspruch 3, dadurch gekennzeichnet, dass ein Reaktionsprodukt der Formel

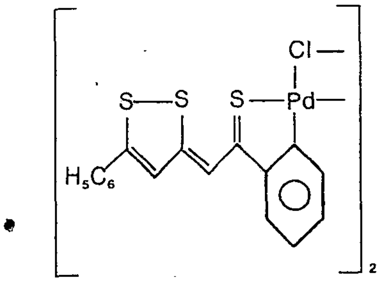

oder

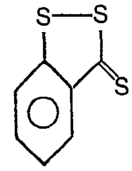

$\cdot 2CuCl_2$ oder

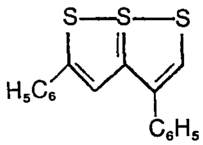

$\cdot 2CuCl_2$

oder

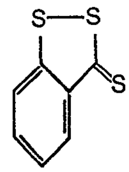

$\cdot$ $2FeCl_3$

mit Lithium umgesetzt wird.

**Claims**

1. A process for the production of organolithium compounds in addition to lithium hydride, characterized in that lithium is reacted in a solvent with an α-olefin or α,ω-diolefin in the presence of a catalyst from a mixture of heterocompounds I to V

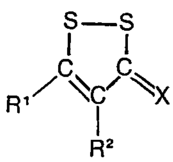

I

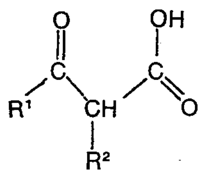

II

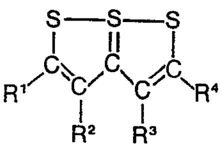

III

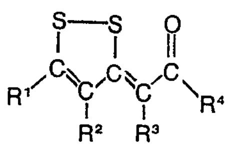

IV

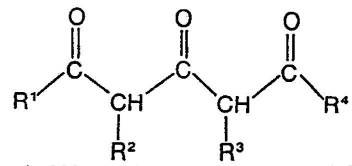

V

wherein X is sulfur or oxygen, $R^1$, $R^2$, $R^3$ and $R^4$ are hydrogen, alkyl, cycloalkyl, aralkyl or aryl groups and/or 2 or more of such groups are closed to form an aliphatic or aromatic ring system with a transition metal compound of metals of subgroups I, II, IV to VII and VIII of the Periodic Table or polycyclic aromatics such as anthracene, naphthalene and biphenyl with a transition metal compound of metals of subgroups I, II, IV to VII and VIII of the Periodic Table.

2. A process according to claim 1, characterized in that adducts between said compounds I to V and the said transition metal compounds, especially copper, iron and palladium halides are isolated and subsequently allowed to act on lithium and olefins and diolefins, respectively.

3. A process according to claim 1, characterized in that a reaction product of the formula

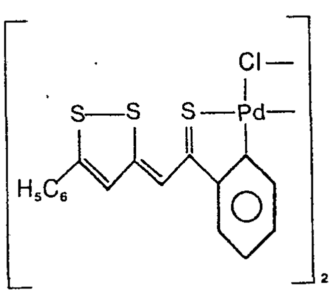

or

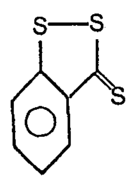

$\cdot 2CuCl_2$ or

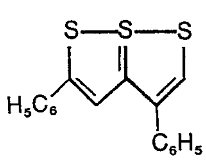

$\cdot 2CuCl_2$

or

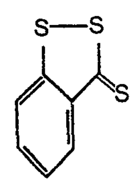

$\cdot$ $2FeCl_3$

is reacted with lithium.

4. A process according to claims 1 to 3, characterized in that the metals are copper, gold, zinc, cadmium, titanium, zirconium, vanadium, niobium, tantalum, chromium, molybdenum, tungsten, manganese, iron, cobalt, nickel, rhutenium, rhodium, palladium, osmium, iridium and platinum.

5. A process according to claims 1 to 4, characterized in that the metals are copper, iron, zinc, palladium, platinum, and rhodium.

6. A process according to claims 1 to 5, characterized in that the reaction is carried out in a cyclic or an open-chain mono- or polyether as the solvent and preferentially in tetrahydrofuran.

7. A process according to claims 1 to 6, characterized in that the reaction is carried out at temperatures between −100 to +100 °C and preferably between −20 and +50 °C.

8. A process according to claims 1 to 7, characterized in that the process is operated at partial pressures of less than 1 to 100 bars.

9. A process according to claims 1 to 8, characterized in that α-olefins of the general formula $CH_2{=}CHR$ wherein R = H, alkyl, aryl, cycloalkyl and aralkyl or α,ω-diolefins of the general formula $CH_2{=}CH\text{-}(CHR)_n\text{-}CH{=}CH_2$ wherein R has the meaning defined above and n = 1 to 6 are used.

10. Adducts between hetero compounds of the general formulas I to V

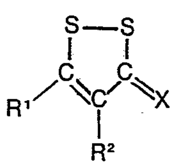 I

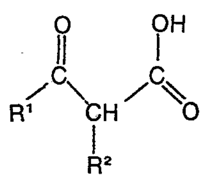 II

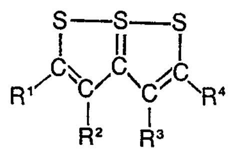 III

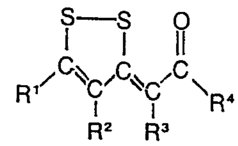 IV

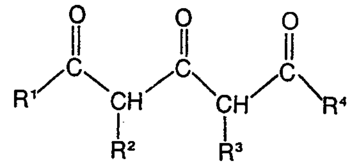 V

wherein X is sulfur or oxygen, $R^1$, $R^2$, $R^3$ and $R^4$ are hydrogen, alkyl, cycloalkyl, aralkyl or aryl groups and/or 2 or more of such groups are closed to form an aliphatic or aromatic ring system and a transition metal compound of metals of subgroups I, II IV to VII and VIII of the Periodic Table.

11. Adducts according to claim 10, characterized in that the metals are copper, gold, zinc, cadmium, titanium, zirconium, vanadium, miobium, tantalum, chromium, molybdenum, tungsten, manganese, iron, cobalt, nickel, rhutenium, rhodium, palladium, osmium, iridium and platinum.

12. Adducts according to claim 10, characterized in that the metals are copper, iron, zinc, palladium, platinum and rhodium.

13. Adducts according to claims 10 to 12 and according to claim 3, characterized in that a reaction product of the formula

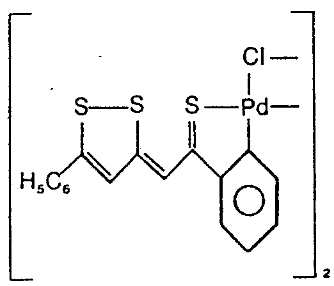

or

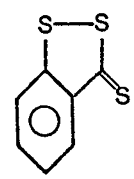 · $2CuCl_2$ or

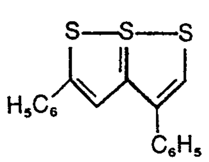 · $2CuCl_2$

or

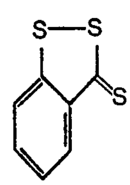 · $2FeCl_3$

is reacted with lithium.

**Revendications**

1. Procédé pour préparer, en plus de l'hydrure de lithium, des composés organiques de lithium, caractérisé en ce qu'on fait réagir du lithium en présence d'un catalyseur formé d'un mélange d'hétérocomposés (I) à (V):

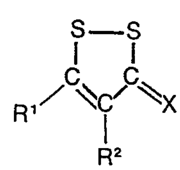 I

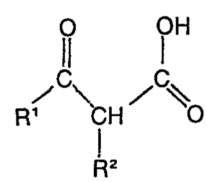 II

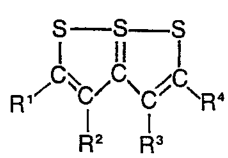 III

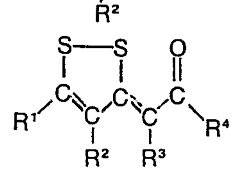 IV

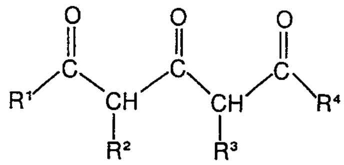

V

dans lesquels X représente le soufre ou l'oxygène, $R^1$, $R^2$, $R^3$ et $R^4$ représentent de l'hydrogène, des radicaux alkyles, cycloalkyles, aralkyles ou aryles et/ou deux ou plusieurs de ces radicaux sont cyclisés pour former un système cyclique aliphatique ou aromatique, avec un composé de métal de transition dérivant des métaux des sous-groupes I, II, IV à VII et VIII du Tableau Périodique ou des hydrocarbures aromatiques polycycliques comme l'anthracène, le naphtalène et le biphényle avec un composé de métal de transition dérivant de métaux des sous-groupes I, II, IV à VII et VIII du Tableau Périodique, dans un solvant, avec une oléfine alpha ou une dioléphine-alpha,gamma.

2. Procédé selon la revendication 1, caractérisé en ce qu'on isole les produits d'addition entre les composés (I) à (V) et les composés cités de métaux de transition, en particulier des halogénures de cuivre, de fer et de palladium et on les fait agir ensuite sur du lithium et des oléfines ou des dioléfines.

3. Procédé selon la revendication 2, caractérisé en ce qu'on fait réagir un produit de réaction de formule:

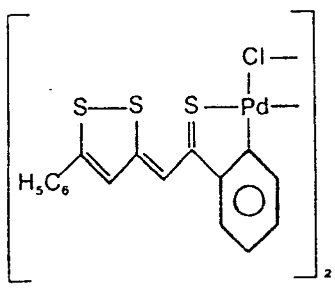

ou

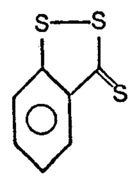

· $2CuCl_2$ ou

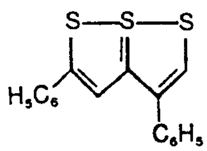

· $2CuCl_2$

ou

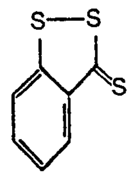

· $2FeCl_3$

avec du lithium.

4. Procédé selon les revendications 1 à 3, caractérisé en ce que les métaux sont le cuivre, l'or, le zinc, le cadmium, le titane, le zirconium, le vanadium, le niobium, le tantale, le chrome, le molybdène, le tungstène, le manganèse, le fer, le cobalt, le nickel, le ruthénium, le rhodium, le palladium, l'osmium, l'iridium et le platine.

5. Procédé selon les revendications 1 à 4, caractérisé en ce que les métaux sont le cuivre, le fer, le zinc, le palladium, le platine et le rhodium.

6. Procédé selon les revendications 1 à 5, caractérisé en ce qu'on effectue la réaction dans un mono- ou polyéther cyclique ou à chaîne ouverte servant de solvant, de préférence dans du tétrahydrofuranne.

7. Procédé selon les revendications 1 à 6, caractérisé en ce qu'on travaille à des températures comprises entre −100 et +100 °C, de préférence entre −20 et +50 °C.

8. Procédé selon les revendications 1 à 7, caractérisé en ce qu'on travaille sous des pressions partielles comprises entre une pression inférieure à 1 et 100 bars.

9. Procédé selon les revendications 1 à 8, caractérisé en ce qu'on fait réagir des oléfines alpha de formule générale $CH_2=CHR$, dans laquelle R représente de l'hydrogène, un groupe alkyle, aryle, cycloalkyle et aralkyle, ou des dioléfines-alpha,oméga de formule générale $CH_2=CH-(CHR)_n-CH=CH_2$, dans laquelle R a le sens précité et n vaut 1 à 6.

10. Produit d'addition entre des hétérocomposés de formule générale (I) à (V):

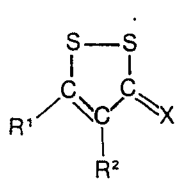

I

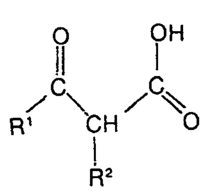

II

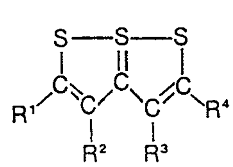

III

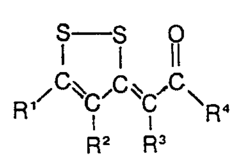

IV

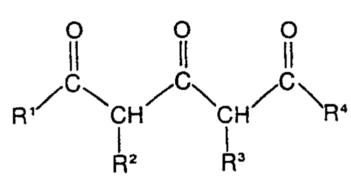

V

dans lesquelles X représentent le soufre ou l'oxygène; $R^1$, $R^2$, $R^3$ et $R^4$ représentent de l'hydrogène ou des radicaux alkyles, cycloalkyles, aralkyles ou aryles, et/ou deux ou plusieurs de ces radicaux sont cyclisés pour former un système cyclique aliphatique ou aromatique, et un composé de métal de transition dérivant des métaux des sous-groupes I, II, IV à VII et VIII du Tableau Périodique.

11. Produit d'addition selon la revendication 10, caractérisé en ce que les métaux sont le cuivre, l'or, le zinc, le cadmium, le titane, le zirconium, le vanadium, le niobium, le tantale, le chrome, le molybdène, le tungstène, le manganèse, le fer, le cobalt, le nickel, le ruthénium, le rhodium, le palladium, l'osmium, l'iridium et le platine.

12. Produit d'addition selon la revendication 10, caractérisé en ce que les métaux sont le cuivre, le fer, le zinc, le palladium, le platine et le rhodium.

13. Produit d'addition selon les revendications 10 à 12 et selon la revendication 3, caractérisé en ce qu'on fait réagir avec du lithium un produit de réaction de formule:

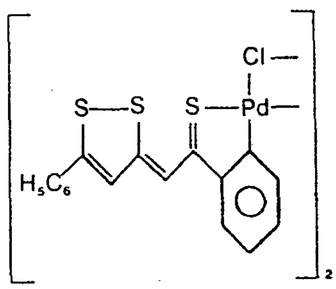

ou

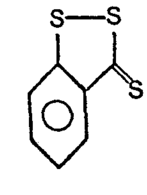

· $2CuCl_2$ ou

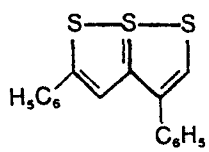

· $2CuCl_2$

ou

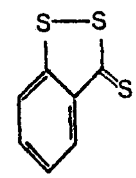

· $2FeCl_3$